# EUROPEAN PATENT APPLICATION

(11) **EP 4 282 338 A1**
(43) Date of publication of application: **29.11.2023**
(21) Application number: 22175706.5
(22) Date of filing: 27.05.2022
(51) Int. Cl.: A61B 6/03, A61B 6/00, A61G 3/00

(54) **MEDICAL VEHICLE AND METHOD FOR OPERATING A MEDICAL VEHICLE**

(71) Applicant: Koninklijke Philips N.V., 5656 AG Eindhoven (NL)
(72) Inventor: JOHNSON, Mark, Thomas, Eindhoven (NL); BUSSA, Nagaraju, Eindhoven (NL); VOGTMEIER, Gereon, 5656AG Eindhoven (NL); NAIK, Sarif, Kumar, 5656AG Eindhoven (NL); LEUSSLER, Christoph, Günther, Eindhoven (NL)
(74) Representative: Philips Intellectual Property & Standards

(57) **Abstract**

The invention relates to a medical vehicle (1) comprising a base vehicle (2) and a compartment (3) with a medical imaging system (4). The compartment (3) is a part of the base vehicle (2) or the compartment (3) is attached to the base vehicle (2). The base vehicle (2) comprises at least one base vehicle sensor (5) and the medical vehicle (1) is configured to obtain sensor readings from the at least one base vehicle sensor (5) during operation of the medical vehicle (1). Said sensor readings are analyzed with regard to adverse conditions for the medical imaging system (4) and feedback is provided based on the analyzed sensor readings. The invention further relates to a corresponding method for operating a medical vehicle (1).

## Description

### FIELD OF THE INVENTION

The invention relates to a medical vehicle comprising a base vehicle and a compartment with a medical imaging system and to a method for operating a medical vehicle.

### BACKGROUND OF THE INVENTION

Vehicle-based medical imaging solutions provide, for example, medical imaging at remote places. During the transporation of the imaging systems, they may be subjected to impact forces caused by the motion of the vehicle and to adverse environmental conditions. Said impact forces or adverse environmental conditions may damage the imaging system such that a re-calibration, an unscheduled maintenance or even a repair is required.

### SUMMARY OF THE INVENTION

It is an object of the present invention to provide a medical vehicle that is configured to assess adverse conditions for a medical imaging system of the medical vehicle while keeping the cost increase of the medical vehicle moderate. It is a further object of the present invention to provide a method for operating a medical vehicle that assesses adverse conditions for the medical imaging system.

The object of the present invention is solved by the subject-matter of the independent claims, wherein further embodiments are incorporated in the dependent claims.

In an aspect of the present invention, a medical vehicle comprising a base vehicle and a compartment with a medical imaging system is provided. Said medical vehicle may offer mobile diagnostic imaging. In particular, the medical vehicle may be configured to transport the medical imaging system, but the medical imaging system may be operated only when the medical vehicle is stopped. Alternatively, or additionally, the medical imaging system may also be operated when the medical vehicle is in motion. Further, the main purpose of the medical vehicle may be the transportation of the medical imaging system, e.g., a medical imaging system on a truck. Alternatively, or additionally, the main purpose of the medical vehicle may be a non-medical one, such as a cruise ship that has a medical imaging system as part of its medical equipment.

The base vehicle is the vehicle transporting the compartment with the medical imaging system, i.e., the base vehicle is the medical vehicle without the compartment with the medical imaging system. As above, the base vehicle may be specifically configured to transport the medical imaging system, such as truck with a medical imaging system, and/or the base vehicle may have a non-medical main purpose, such as a cruise ship.

The compartment is a part of the base vehicle or is attached to the base vehicle. In other words, the compartment may be a virtual compartment defined by a volume of an existing compartment of the base vehicle or may comprise physical walls. In the latter case, the compartment may be permanently attached to the base vehicle or may be detachable such that it can be placed separately from the base vehicle. The compartment may be just large enough to fit the medical imaging system, but it may also provide extra space next to the medical imaging system, e.g., for medical staff.

The base vehicle comprises at least one base vehicle sensor. Said base vehicle sensor may be used to assess adverse conditions for the medical imaging system.

The medical vehicle is configured to obtain sensor readings from the at least one base vehicle sensor during operation of the medical vehicle. In this context, operation may refer to travel of the medical vehicle but may also encompass a stationary medical vehicle, e.g., with the engine running or with the ignition turned on. If the base vehicle sensor is already present in the base vehicle, there is no need to add an extra sensor, which may result in considerable cost savings.

The medical vehicle is further configured to analyze the sensor readings with regard to adverse conditions for the medical imaging system and to provide feedback based on the analyzed sensor readings. The provided feedback may remedy said adverse conditions. And if the base vehicle sensor is already present in the medical imaging system, said assessment of adverse conditions and feedback to remedy the adverse conditions may be obtained at a very low cost.

According to an embodiment, the medical vehicle is a truck, a train, a plane, a helicopter, an autonomous flight object and/or a ship. In all of said vehicles, a medical imaging system may be installed and may be transported. Adverse conditions may stem from, e.g., potholes, corners, hills, turbulences, waves or environmental conditions and may be assessed using the sensor readings from the at least one base vehicle sensor.

According to an embodiment, the medical imaging system is a magnetic resonance imaging (MRI), a computed tomography (CT), a digital X-ray radiogrammetry (DXR), a single-photon emission computed tomography (SPECT), a positron emission tomography (PET), a dark field X-ray imaging, a small dedicated medical imaging and/or an ultrasound system. Each of these systems is very sensitive to adverse conditions such as forces acting upon it and hence an assessment of the adverse conditions is very valuable.

According to an embodiment, the at least one base vehicle sensor is a vehicle navigation system, a compass, a camera, a radar, a lidar, a speedometer, a wind sensor, a wave sensor, an acceleration sensor, an inertial measurement unit (IMU), a motion control sensor, an engine control sensor, a vibration sensor, an electrical sensor such as a voltage or current sensor, a temperature sensor, a humidity sensor and/or an electromagnetic field sensor. As an example, the vehicle navigation system may provide information on road conditions ahead, such as curves, inclinations or even road bumps, wherein said information may, for example, also be used for driver assistance systems and/or for autonomous driving. The camera, radar and/or lidar may also detect road conditions ahead for a land vehicle or water conditions for a sea vehicle. The engine control sensor may provide, e.g., information on the r.p.m. of the engine, which may be used to predict resonant vibrations. The vibration sensor may detect vibrations of the base vehicle and/or of the compartment. The electrical sensor may measure the electric power supply to the compartment and/or to the medical imaging system. Further, the temperature sensor, the humidity sensor and/or the electromagnetic field sensor may measure the temperature, humidity and/or electromagnetic fields, respectively, outside of the vehicle and/or inside the vehicle, in particular inside the compartment.

According to an embodiment, the adverse conditions for the medical imaging system may be impact forces on the compartment, adverse environmental conditions in the compartment, power outages affecting the medical imaging system and high intensity electromagnetic fields affecting the medical vehicle. In this context, impact force may be any force other than the gravitational force acting in a predetermined downwards direction. In particular, impact forces may be forces due to an acceleration of the compartment housing the medical imaging system. As examples, impact forces may be forces caused by bumps in the road, may be centrifugal forces when the medical vehicle changes direction or may be caused by tilting the compartment, such that the direction of the gravitational force changes. The environmental conditions may be, e.g., temperature, humidity and/or atmospheric pressure and become adverse environmental conditions when said parameters are outside a predetermined range. Power outages affecting the medical imaging system may, for example, affect the air conditioning of the compartment and/or medical imaging system, and, in turn, the environmental conditions, or may affect electronic components that may lose, e.g., calibration settings. Power outages may also affect software updates of the medical imaging system that have been scheduled to be performed during transportation, may interrupt the flow of wireless satellite data, may lead to a re-booting of the medical imaging system and/or may affect remote monitoring and service of the medical imaging system. Further, high intensity electromagnetic fields caused, e.g., by transformers or by radio antennas, may also affect the medical imaging system.

According to an embodiment, the medical vehicle is configured such that analyzing the sensor readings comprises predicting the adverse conditions for the medical imaging system. As an example, data from the vehicle navigation system indicating road bumps and curves ahead together with the current vehicle speed known from the speedometer may be used to predict impact forces. As another example, known road inclinations may be used to predict impact forces due to tilting the compartment. As yet another example, altitude information on a map together with the predicted vehicle route may be used to predict atmospheric pressure. As yet another example, wave sensors of a ship may be used to predict impact forces. If said predicted adverse conditions exceed a predetermined adverse condition prediction threshold, the feedback is provided to a vehicle management system of the medical vehicle and/or to a compartment management system and comprises control commands to adapt vehicle settings and/or compartment settings. As an example, the speed of the vehicle may be reduced such that the impact forces due to road bumps and/or curves are reduced. Also, the acceleration and/or deceleration of the vehicle may be limited in order to reduce the impact forces. As other examples, settings of a suspension system of the vehicle may be adjusted to absorb at least some of the impact forces and/or hydraulic systems of the vehicle may be set to elevate a ground clearance of the vehicle. Alternatively, or additionally, settings of an anchoring system which anchors the medical imaging system to the compartment, e.g., the strength of shock absorbers therein, may be adjusted to absorb at least some of the impact forces. Said anchoring system may have several modes such as a locking mode, a suspension mode and a damping mode. In the locking mode, the medical imaging system is locked to the compartment, which prevents large scale movements of the medical imaging system during transportation. In the suspension mode, there is a suspension such as a spring suspension that allows limited motion of the medical imaging system and absorbs some of the impact forces that act on the compartment. And in the damping mode, said suspension is complemented by a damping system such as shock absorbers to prevent oscillations. Depending on the predicted impact forces on the compartment, the mode that leads to the least damage to the medical imaging system is selected. If both the suspension system of the vehicle and the anchoring system are adjusted, a control unit may be used to coordinate the adjustments of these systems, in particular in order to avoid overcompensation and to avoid resonant oscillations of the medical imaging system. As yet another example, a pressurization system may be activated if high altitudes and hence low atmospheric pressures are predicted.

According to an embodiment, the medical vehicle is configured such that analyzing the sensor readings comprises detecting the adverse conditions for the medical imaging system. This may include detecting adverse conditions that have been predicted but could not be fully remedied by adapting the vehicle settings and/or compartment settings. Detecting the adverse conditions may also include detecting adverse conditions that have not been predicted and/or could not be predicted.

According to an embodiment, if the detected adverse conditions exceed a predetermined first adverse condition threshold, the feedback is provided to a vehicle management system of the medical vehicle and/or a compartment management system and comprises control commands to adapt vehicle settings and/or compartment settings. As an example, the detected adverse conditions may be impact forces detected by the inertial measurement unit, by the motion control sensor, by the vibration sensor or by an analysis of the actual route taken, based on input from the vehicle navigation system. The vehicle management system may be, e.g., an on-board vehicle computer and the adaptation may be a control of the vehicle's speed and/or an adaptation of an active suspension of the vehicle. Controlling the vehicle's speed may be performed, e.g., via an adaptive cruise control or via a speed limit presented to the operator, e.g., driver. Once the vehicle has slowed down, the impact forces acting on the medical imaging system and on the vehicle will also be reduced Also, by performing an adaptation of the active suspension of the vehicle, the active suspension parameters are reconfigured such that the forces acting on the imaging system are reduced. The same holds true for adaptations of the compartment settings that may include, e.g., settings of the anchoring system that anchors the medical imaging system to the compartment. In conclusion, the risk for damage to the medical imaging system is reduced and even less experienced drivers may safely operate the vehicle. As another example, the detected adverse conditions may be adverse environmental conditions such as excessive heat. In this case, the settings of an environmental conditioning system, which may belong to the vehicle settings and/or to the compartment settings, may be adapted in order to cool the medical imaging system.

According to an embodiment, if the detected adverse conditions exceed a predetermined second adverse condition threshold, the feedback is an alert and/or a control command for re-calibration and/or maintenance of the medical imaging system. Said second adverse condition threshold may be higher than the first adverse condition threshold, such that only those adverse conditions are object to re-calibration and/or maintenance that could not be prevented by adapting the vehicle settings and/or compartment settings. The alert for re-calibration and/or maintenance may be provided to a user interface such that, e.g., an operator of the medical vehicle is notified to have the medical imaging system recalibrated and/or maintained. The control command may automatically start the execution of a re-calibration or maintenance action, e.g., when the imaging system is started. Further, if, e.g., a high temperature was detected, the control command may comprise a waiting time to delay the execution of the re-calibration until the entire medical imaging system has cooled down and stable imaging conditions have been achieved.

According to an embodiment, if the detected adverse conditions exceed a predetermined third adverse condition threshold, the feedback is an alert for inspection and/or repair of the medical imaging system. Said third adverse condition threshold may be higher than the second adverse condition threshold. The alert for inspection and/or repair of the medical imaging system may be provided to a user interface such that, e.g., the operator of the medical vehicle is notified to have the medical imaging system inspected and, if necessary, repaired. The feedback may also comprise a control command that may automatically run a predetermined set of self-tests of the medical imaging system and then provide further feedback, e.g., a further alert.

According to an embodiment, the vehicle settings that are to be adapted are the vehicle speed, the vehicle route, vehicle suspension settings, and/or environmental conditioning settings. With a reduced vehicle speed, the impact forces due to road bumps or curves are reduced. Also, for example, if the vehicle route contains rough roads, a change in vehicle route may guide the medical vehicle on smoother roads. Changing vehicle suspension settings, e.g., the strength of shock absorbers, may also reduce the impact forces, in particular, in combination with a reduced vehicle speed. And adapting environmental condition settings may be performed to control an environmental conditioning system that may, besides controlling the temperature of the compartment and/or medical imaging system, control humidity of the compartment and/or medical imaging system.

In another aspect of the invention, a method for operating a medical vehicle according to the above description is provided. The method comprises obtaining sensor readings from the at least one base vehicle sensor during operation of the medical vehicle, analyzing the sensor readings with regard to adverse conditions for the medical imaging system, and providing feedback based on the analyzed sensor readings. In particular, the sensor readings may be analyzed to assess adverse conditions for the medical imaging system and the provided feedback may remedy said adverse conditions. And if the at least one base vehicle sensor is already present in the medical vehicle, said assessment of adverse conditions and feedback to remedy the adverse conditions may be obtained at a very low cost. Further advantages are provided in the above description.

According to an embodiment, analyzing the sensor readings comprises predicting the adverse conditions for the medical imaging system. If the predicted adverse conditions exceed a predetermined adverse condition prediction threshold, the feedback is provided to a vehicle management system of the medical vehicle and/or compartment management system and comprises control commands to adapt vehicle settings and/or compartment settings. Further details, examples and advantages are provided in the above description.

According to an embodiment, analyzing the sensor readings comprises detecting the adverse conditions for the medical imaging system. If the detected adverse conditions exceed a predetermined first adverse condition threshold, the feedback is provided to the vehicle management system of the medical vehicle and/or compartment management system and comprises control commands to adapt vehicle settings and/or compartment settings. If the detected adverse conditions exceed a predetermined second adverse condition threshold, which may be higher than the first adverse condition threshold, the feedback is an alert and/or a control command for re-calibration and/or maintenance of the medical imaging system. And if the detected adverse conditions exceed a predetermined third adverse condition threshold, which may be higher than the second adverse condition threshold, the feedback is an alert for inspection and/or repair of the medical imaging system. Further details, examples and advantages are provided in the above description.

According to an embodiment, if the feedback is an alert for re-calibration, maintenance, inspection and/or repair and/or a control command for re-calibration and/or maintenance, the re-calibration, maintenance, inspection and/or repair of the medical imaging system is performed at a destination of the medical vehicle, at a start of the medical imaging system and/or when the detected adverse conditions fall below a predetermined fourth adverse condition threshold. The fourth adverse condition threshold may be lower than the first adverse condition threshold. Further details, examples and advantages are provided in the above description.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematical side view of a medical vehicle; and
Fig. 2 shows a flowchart of a method for operating a medical vehicle.

### DETAILED DESCRIPTION OF EMBODIMENTS

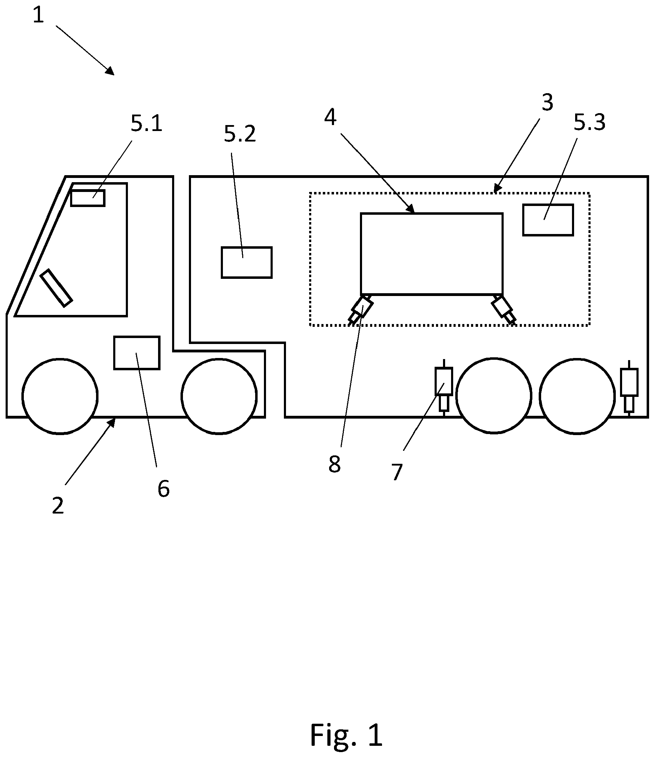

Fig. 1 shows a schematical side view of a medical vehicle 1. The medical vehicle 1 is depicted as a truck, but may as well be a train, a plane, a helicopter, an autonomous flight object or a ship.

The medical vehicle 1 comprises a base vehicle 2 and a compartment 3 with a medical imaging system 4. The compartment 3 may be a part of the base vehicle 2 or may be attached to the base vehicle 2. The medical imaging system 4 may be a magnetic resonance imaging (MRI), a computed tomography (CT), a digital X-ray radiogrammetry (DXR), a single-photon emission computed tomography (SPECT), a positron emission tomography (PET), a dark field X-ray imaging, a small dedicated medical imaging and/or an ultrasound system.

The base vehicle 2 comprises a plurality of base vehicle sensors 5, for example a camera 5.1, an inertial measurement unit 5.2 and a temperature sensor 5.3. Other base vehicle sensors 5 may be a vehicle navigation system, a compass, a radar, a lidar, a speedometer, a wind sensor, a wave sensor, an acceleration sensor, a motion control sensor, an engine control sensor, a vibration sensor, an electrical sensor, a humidity sensor and an electromagnetic field sensor.

The base vehicle 2 further comprises a vehicle management system 6 that is configured to adapt and/or control vehicle settings. Moreover, the base vehicle 2 comprises an active suspension 7 of the vehicle and an anchoring system 8 which anchors the medical imaging system 4 to the compartment 3.

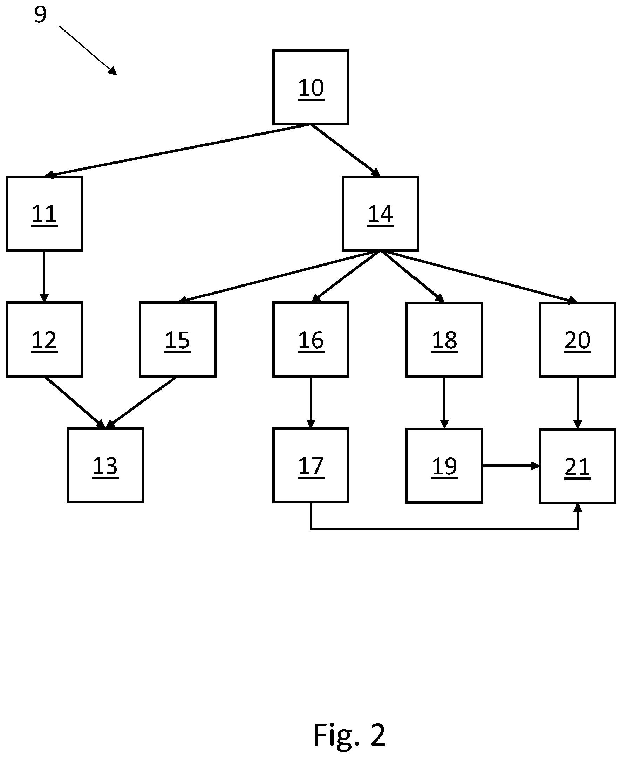

A method 9 for operating the medical vehicle 1 is shown in Fig. 2.

First, sensor readings are obtained 10 from the base vehicle sensors 5. Said sensor readings are then analyzed, for example by a computing unit that is not shown here.

Said analysis comprises a prediction of adverse conditions 11 for the medical imaging system 4. As an example, the camera 5.1 may detect a bumpy and/or curvy road ahead and together with readings of a speedometer, impact forces on the medical imaging system 4 are predicted. As an alternative or in addition to the camera 5.1, the upcoming road conditions may be assessed by the radar and/or the lidar. If said predicted adverse conditions exceed a predetermined adverse condition prediction threshold 12, control commands are provided 13 to the vehicle management system 6 and/or to a compartment management system (not shown here) to adapt vehicle settings and/or compartment settings. In the example of the bumpy road, such control commands may be a reduction of the vehicle speed as well as a softening of the active suspension 7 and of shock absorbers of the anchoring system 8.

The analysis of the sensor readings further comprises detecting adverse conditions 14 for the medical imaging system 4. If the detected adverse conditions exceed a predetermined first adverse condition threshold 15, control commands are provided 13 to the vehicle management system and/or to the compartment management system to adapt vehicle settings and/or compartment setting, similarly to the case when the predicted adverse conditions exceed the adverse condition prediction threshold 12.

If the detected adverse conditions exceed a predetermined second adverse condition threshold 16, which may be higher than the first adverse condition threshold, an alert and/or a control command for re-calibration and/or maintenance 17 of the medical imaging system 4 are provided.

If the detected adverse conditions exceed a predetermined third adverse condition threshold 18, which may be higher than the second adverse condition threshold, an alert and/or a control command for inspection and/or repair 19 of the medical imaging system 4 are provided.

Finally, if the detected adverse conditions fall below a predetermined fourth adverse condition threshold 20, which may be lower than the first adverse condition threshold, the re-calibration, maintenance, inspection and/or repair of the medical imaging system 4, given by the control commands for re-calibration and/or maintenance 17 and/or the control commands for inspection and/or repair 19 are executed 21.

Alternatively, or additionally, the control commands for re-calibration and/or maintenance 17 and/or the control commands for inspection and/or repair 19 may be executed when the medical imaging system 4 has reached its destination and/or upon a start of the medical imaging system 4.

In conclusion, adverse conditions for the medical imaging system 4 are assessed and remedied and/or accounted for, such that the medical imaging system 4 may always operate according to its specifications.

While the invention has been illustrated and described in detail in the drawing and foregoing description, such illustration and description are to be considered illustrative or exemplary and not restrictive; the invention is not limited to the disclosed embodiments. In particular, several embodiments may be combined to provide optimal limitation of gyroscopic forces.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: medical vehicle
- 2: base vehicle
- 3: compartment
- 4: medical imaging system
- 5: base vehicle sensors
- 5.1: camera
- 5.2: inertial measurement unit
- 5.3: temperature sensor
- 6: vehicle management system
- 7: active suspension
- 8: anchoring system
- 9: method
- 10: obtaining sensor readings
- 11: predicting adverse conditions
- 12: exceeding adverse condition prediction threshold
- 13: providing control commands
- 14: detecting adverse conditions
- 15: exceeding first adverse condition threshold
- 16: exceeding second adverse condition threshold
- 17: providing control commands for re-calibration and/or maintenance
- 18: exceeding third adverse condition threshold
- 19: providing control commands for inspection and/or repair
- 20: falling below fourth adverse condition threshold
- 21: execution of control commands

## Claims

1. Medical vehicle comprising a base vehicle (2) and a compartment (3) with a medical imaging system (4), wherein the compartment (3) is a part of the base vehicle (2) or the compartment (3) is attached to the base vehicle (2), wherein the base vehicle (2) comprises at least one base vehicle sensor (5) and wherein the medical vehicle (1) is configured to
obtain sensor readings from the at least one base vehicle sensor (5) during operation of the medical vehicle (1);
analyze the sensor readings with regard to adverse conditions for the medical imaging system (4); and
provide feedback based on the analyzed sensor readings.

2. Medical vehicle according to claim 1, wherein the base vehicle (2) is a truck, a train, a plane, a helicopter, an autonomous flight object and/or a ship.

3. Medical vehicle according to claim 1 or 2, wherein the medical imaging system (4) is a magnetic resonance imaging, MRI, a computed tomography, CT, a digital X-ray radiogrammetry, DXR, a single-photon emission computed tomography, SPECT, a positron emission tomography, PET, a dark field X-ray imaging, a small dedicated medical imaging and/or an ultrasound system.

4. Medical vehicle according to any of claims 1 to 3, wherein the at least one base vehicle sensor (5) is at least one out of a group, the group consisting of a vehicle navigation system, a compass, a camera (5.1), a radar, a lidar, a speedometer, a wind sensor, a wave sensor, an acceleration sensor, an inertial measurement unit (5.2), a motion control sensor, an engine control sensor, a vibration sensor, an electrical sensor, a temperature sensor (5.3), a humidity sensor and an electromagnetic field sensor.

5. Medical vehicle according to any of claims 1 to 4, wherein the adverse conditions for the medical imaging system (4) are at least one out of a group, the group consisting of impact forces on the compartment (3), adverse environmental conditions in the compartment (3), power outages affecting the medical imaging system (4) and high intensity electromagnetic fields affecting the medical vehicle (1).

6. Medical vehicle according to any of claims 1 to 5, wherein the medical vehicle (1) is configured such that analyzing the sensor readings comprises predicting the adverse conditions for the medical imaging system (4), and if the predicted adverse conditions exceed a predetermined adverse condition prediction threshold, the feedback is provided to a vehicle management system (6) of the medical vehicle (1) and/or compartment management system and comprises control commands to adapt vehicle settings and/or compartment (3) settings.

7. Medical vehicle according to any of claims 1 to 6, wherein the medical vehicle (1) is configured such that analyzing the sensor readings comprises detecting the adverse conditions for the medical imaging system (4).

8. Medical vehicle according to claim 7, wherein, if the detected adverse conditions exceed a predetermined first adverse condition threshold, the feedback is provided to the vehicle management system (6) of the medical vehicle (1) and/or compartment management system and comprises control commands to adapt vehicle settings and/or compartment (3) settings.

9. Medical vehicle according to claim 7 or 8, wherein, if the detected adverse conditions exceed a predetermined second adverse condition threshold, the feedback is an alert and/or a control command for re-calibration and/or maintenance of the medical imaging system (4).

10. Medical vehicle according to any of claims 7 to 9, wherein, if the detected adverse conditions exceed a predetermined third adverse condition threshold, the feedback is an alert for inspection and/or repair of the medical imaging system (4).

11. Medical vehicle according to claim 6 or any of claims 8 to 10, wherein the vehicle settings to be adapted are at least one out of a group, the group consisting of vehicle speed, vehicle route, vehicle suspension settings, and environmental conditioning settings.

12. Method for operating a medical vehicle (1) according to any of claims 1 to 11, comprising:
obtaining sensor readings from the at least one base vehicle sensor (5) during operation of the medical vehicle (1);
analyzing the sensor readings with regard to adverse conditions for the medical imaging system (4); and
providing feedback based on the analyzed sensor readings.

13. Method according to claim 12, wherein the step of analyzing the sensor readings comprises predicting the adverse conditions for the medical imaging system (4), and if the predicted adverse conditions exceed a predetermined adverse condition prediction threshold, the feedback is provided to a vehicle management system (6) of the medical vehicle (1) and/or compartment management system and comprises control commands to adapt vehicle settings and/or compartment (3) settings.

14. Method according to claim 12 or 13, wherein the step of analyzing the sensor readings comprises detecting the adverse conditions for the medical imaging system (4), and
if the detected adverse conditions exceed a predetermined first adverse condition threshold, the feedback is provided to the vehicle management system (6) of the medical vehicle (1) and/or compartment management system and comprises control commands to adapt vehicle settings and/or compartment (3) settings;
if the detected adverse conditions exceed a predetermined second adverse condition threshold, the feedback is an alert and/or a control command for re-calibration and/or maintenance of the medical imaging system (4); and/or
if the detected adverse conditions exceed a predetermined third adverse condition threshold, the feedback is an alert for inspection and/or repair of the medical imaging system (4).

15. Method according to claim 14, wherein, if the feedback is an alert for re-calibration, maintenance, inspection and/or repair and/or a control command for re-calibration and/or maintenance, the re-calibration, maintenance, inspection and/or repair of the medical imaging system (4) is performed at a destination of the medical vehicle (1) and/or when the detected adverse conditions fall below a predetermined fourth adverse condition threshold.
